(19) Europäisches Patentamt
European Patent Office
Office européen des brevets

(11) Numéro de publication: **0 516 802 B1**

(12) **FASCICULE DE BREVET EUROPEEN**

(45) Date de publication de fascicule du brevet: **22.03.95**

(51) Int. Cl.$^6$: **C07C 321/14**, C07C 321/18, C10M 135/22

(21) Numéro de dépôt: **92902163.2**

(22) Date de dépôt: **17.12.91**

(86) Numéro de dépôt internationale :
**PCT/FR91/01026**

(87) Numéro de publication internationale :
**WO 92/12126 (23.07.92 92/19)**

(54) **NOUVEAUX COMPOSES SULFURES, LEUR PREPARATION ET LEUR UTILISATION COMME ADDITIFS POUR HUILES LUBRIFIANTES.**

(30) Priorité: **28.12.90 FR 9016438**

(43) Date de publication de la demande:
**09.12.92 Bulletin 92/50**

(45) Mention de la délivrance du brevet:
**22.03.95 Bulletin 95/12**

(84) Etats contractants désignés:
**BE DE DK ES GB IT NL SE**

(56) Documents cités:

**CHEMISCHE BERICHTE, vol. 91, 1958, pages 996-1005, Weinheim, DE; F. FEHER et al: "Zur Kenntnis der Thiaalkane"**

(73) Titulaire: **INSTITUT FRANCAIS DU PETROLE**
**4, avenue de Bois Préau**
**F-92502 Rueil-Malmaison (FR)**

(72) Inventeur: **ABERKANE, Ourida**
**8, Boucle de la Seille**
**F-57290 Fameck (FR)**

Inventeur: **BORN, Maurice**
**72, rue du Vieux-Pont**
**F-92000 Nanterre (FR)**
Inventeur: **MIELOSZYNSKI, Jean-Luc**
**286, rue Pont-à-Mousson**
**F-57158 Montigny-les-Metz (FR)**
Inventeur: **PAOUER, Daniel**
**Résidence Minerve,**
**2, rue d'Anvers**
**F-54500 Vandoeuvre (FR)**
Inventeur: **PARC, Guy**
**27, rue du Château**
**F-92500 Rueil-Malmaison (FR)**

(74) Mandataire: **Andreeff, François et al**
**INSTITUT FRANCAIS DU PETROLE**
**4, avenue de Bois-Préau**
**F-92506 Rueil-Malmaison Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 516 802 B1

Rank Xerox (UK) Business Services
(3.10/3.09/3.3.3)

## Description

La présente invention concerne de nouveaux composés sulfurés, leur preparation et leur utilisation comme additifs pétroliers, notamment comme additifs antiusure et extême-pression pour huiles lubrifiantes.

L'utilisation d'additifs antiusure et extrême pression notamment dans les huiles moteurs, les fluides de transmission, les fluides hydrauliques se pratique depuis plusieurs décennies. de nombreux types d'additifs ont ainsi été développés et plusieurs d'entre eux ont permis de réduire très sensiblement la détérioration des mécanismes et ont, de ce fait, permis de prolonger leur durée de vie.

Parmi les nombreux additifs antiusure et etrême-pression qui ont été étudiés, les dialkyl- et diaryldithiophosphates et les dialkyldithiocarbamates métalliques (ceux de zinc notamment), les alkylthiophosphates, le tricrésylphosphate, le didodécylphosphate, les terpènes sulfurés, l'huile de spermacéti soufrée et divers composés chlorés se sont montrés les plus actifs et ont connu un développement industriel important. Certains d'entre eux sont décrits dans les brevets US-A-2.364.283, 2.364.284, 2.365.938, 2.410.650, 2.438.876 et 3.190.833. Il s'agit généralement de composés renfermant des hétéroatomes tels que le soufre et le phosphore, soit seuls (par exemple le tricrésylphosphate, les terpènes soufrés, les dithiocarbamates), soit en association (par exemple des dialkyldithiophosphates métalliques, des alkylthiophosphates).

Parmi les composés soufrés, certains peuvent renfermer des fonctions chimiques conférant au composé, dans son utilisation comme additif, des propriétés physico-chimiques ou des performances mécaniques améliorées. Il s'agit entre autres des fonctions phénol, nitrile, sulfoxyde, xanthate, etc. A cet égard, on peut citer les brevets américains US-A-3.434.852, 3.984.336 et 3.994.923.

La quantité de soufre contenue dans la molécule de ces composés soufrés est généralement fixée par la stoechiométrie des réactions mises en oeuvre lors de leur synthèse, ce que leur confère des propriétés antiusure et extrême-pression sur lesquelles on ne peut exercer aucune modification.

Or, on a maintenant découvert de nouveaux composés soufrés, utilisables comme additifs pour lubrifiants, pour lesquels il est possible de modifier la proportion de soufre qu'ils contiennent dans leur molécule et donc de moduler à volonté leurs propriétés antiusure et extrême-pression.

Les composés sulfurés de l'invention peuvent être définis par le fait qu'ils répondent à la formule générale :

$$\{[R\text{-}S_x\text{-}(CH_2)_n\text{-}]_z\ CH_{3-z}\text{-}\ (CH_2)_p\ \text{-}\}_2\ S_y \qquad (I)$$

dans laquelle R représente un radical hydrocarboné monovalent de 1 à 30 atomes de carbone, éventuellement fonctionnalisé ; x est un nombre égal ou supérieur à 1 ; z est un nombre entier égal à 1 ou 2; lorsque z = 1, n est un nombre entier de 0 à 3 et p est un nombre entier de 0 à 3, avec n + p entier de 0 à 3 ; lorsque z = 2, n est un nombre entier de 1 à 3 et p un nombre entier de 0 à 3 ; et y est un nombre égal ou supérieur à 1; au moins un des nombres x et y étant strictement supérieur à 1.

Par radical fontionnalisé, on entend, pour la définition de R, un radical comportant au moins un hétéroatome monovalent pendant, tel que par exemple au moins un atome de chlore, et/ou au moins un hétéroatome divalent dans la chaîne hydrocarbonée, tel que par exemple au moins un atome d'oxygène ou de soufre, et/ou au moins une fonction chimique telle que acide carboxylique, aldéhyde, cétone, nitrile, hydroxyle ou époxyde.

Par ailleurs, il doit être entendu que les composés de l'invention peuvent consister en des mélanges de plusieurs molécules répondant à la formule (I) dans lesquelles la valeur de x et la valeur de y seront différentes d'une molécule à l'autre. Ainsi, on peut considérer que dans la formule (I) les nombres x et y représentent des valeurs statistiques.

Dans la formule (I) des composés sulfurés de l'invention, plus particulièrement R représente un radical alkyle renfermant par exemple de 1 à 10 atomes de carbone ou un radical alkényle renfermant par exemple de 2 à 10 atomes de carbone, par exemple un radical tertiobutyle $(CH_3)_3C$- ou un radical méthallyle $CH_2=C(CH_3)_3\text{-}CH_2$-; x a une valeur moyenne de 1 à 3 ; z=1, avec n + p de 1 à 3, et y a une valeur moyenne de 1 à 3.

Comme exemples de tels composés sulfurés selon l'invention, on peut citer les composés de formules :

$[t.Bu\text{-}S\text{-}(CH_2)_2\text{-}]_2\ S_2$ ; $[t.Bu\text{-}S\text{-}(CH_2)_3\text{-}]_2\ S_2$ ;
$[t.Bu\text{-}S\text{-}(CH_2)_2\text{-}]_2\ S_3$ ; $[t.Bu\text{-}S\text{-}(CH_2)_3\text{-}]_2\ S_3$ ;
$[t.Bu\text{-}S_2\text{-}(CH_2)_2\text{-}]_2\ S$ ; $[t.Bu\text{-}S_2\text{-}(CH_2)_3\text{-}]_2\ S$ ;
$[t.Bu\text{-}S_2\text{-}(CH_2)_2\text{-}]_2\ S_2$ ; $[t.Bu\text{-}S_2\text{--}(CH_2)_3\text{-}]_2 S_2$ ;
$[t.Bu\text{-}S_2\text{-}(CH_2)_2\text{-}]_2\ S_3$ ; $[t.Bu\text{-}S_2\text{-}(CH_2)_3\text{-}]_2 S_3$ ;

[t.Bu-$S_3$-$(CH_2)_2$-]$_2$S ; [t.Bu- $S_3$-$(CH_2)_3$-]$_2$S ;
[t.Bu-$S_3$-$(CH_2)$-]$_2$$S_2$ ; [t.Bu-$S_3$-$(CH_2)_3$-]$_2$$S_2$ ;
[t.Bu-$S_3$-$(CH_2)_2$-]$_2$$S_3$ ; [t.Bu-$S_3$-$(CH_2)_3$-]$_2$$S_3$ ;
[méthallyl-S-$(CH_2)_2$-]$_2$ $S_2$ ; [méthallyl-S-$(CH_2)_3$-]$_2$ $S_2$ ;
[méthallyl-S-$(CH_2)_2$-]$_2$ $S_3$ ; [méthallyl-S-$(CH_2)_3$-]$_2$ $S_3$ ;
[méthallyl-$S_2$-$(CH_2)_2$-]$_2$ S ; [méthallyl-$S_2$-$(CH_2)_3$-]$_2$ S ;
[méthallyl-$S_2$-$(CH_2)_2$-]$_2$S2 ; [méthallyl-$S_2$-$CH_2)_3$-]$_2$$S_2$ ;
[méthallyl-$S_2$-$(CH_2)_2$-]$_2$$S_3$ ; [méthallyl-$S_2$-$(CH_2)_3$-]$_2$$S_3$ ;
[méthallyl-$S_3$-$(CH_2)_2$-]$_2$S ; [méthallyl-$S_3$-$(CH_2)_3$-]$_2$S ;
[méthallyl-$S_3$-$(CH_2)_2$-]$_2$$S_2$ ; [méthallyl-$S_3$-$(CH_2)_3$-]$_2$$S_2$ ;
[méthallyl-$S_3$-$(CH_2)_2$-]$_2$$S_3$ et [méthallyl-$S_3$-$(CH_2)_3$-]$_2$$S_3$ ;

On peut également considérer plus particulièrement les composés répondant à la forme générale (I) dans laquelle R représente un radical alkyle renfermant par exemple de 1 à 10 atomes de carbone ou un radical alkényle renfermant par exemple de 2 à 10 atomes de carbone, plus particulièrement un radical tertio-butyle ou méthallyle; x a une valeur moyenne de 1 à 3, z est égal à 2 avec n = 1 et p = 0, et y a une valeur moyenne de 1 à 3.

Comme exemples de tels composés selon l'invention, on peut citer ceux qui répondent aux formules suivantes :

[(t.Bu-S-CH-$_2$-)$_2$ CH-]$_2$ $S_2$
[(t.Bu-S-$CH_2$-)$_2$ CH-]$_2$ $S_3$
[(t.Bu-$S_2$-$CH_2$-)$_2$ CH-]$_2$ S
[(t.Bu-$S_2$-$CH_2$-)$_2$ CH-]$_2$ $S_2$
[(t.Bu-$S_2$-$CH_2$-)$_2$ CH-]$_2$ $S_3$
[(t.Bu-$S_3$-$CH_2$-)$_2$ CH-]$_2$ S
[(t.Bu-$S_3$-$CH_2$-)$_2$ CH-]$_2$ $S_2$
[(t.Bu-$S_3$-$CH_2$-)$_2$ CH-]$_2$ $S_3$
[(méthallyl-S-$CH_2$-)$_2$ CH-]$_2$ $S_2$
[(méthally-S-$CH_2$-)$_2$ CH-]$_2$ $S_3$
[(méthallyl-$S_2$-$CH_2$-)$_2$ CH-]$_2$ S
[(méthallyl-$S_2$-$CH_2$-)$_2$ CH-]$_2$ $S_2$
[(méthallyl-$S_2$-$CH_2$-)$_2$ CH-]$_2$ $S_3$
[(méthallyl-$S_3$-$CH_2$-)$_2$ CH-]$_2$ S
[(méthallyl-$S_3$-$CH_2$-)$_2$ CH-]$_2$ $S_2$ et
[(méthallyl-$S_3$-$CH_2$-)$_2$ CH-]$_2$ $S_3$

Dans les formules ci-dessus t.Bu désigne en abrégé le radical tertio-butyle.

Les composés sulfurés de l'invention peuvent être préparés par des procédés tels qu'illustrés dans les exemples que l'on trouvera plus loin.

Les composés sulfurés de l'invention ont en général une teneur en soufre assez élevée, qui varie suivant le nombre d'atomes de soufre des enchaînements -$S_x$- et -$S_y$- présents dans leur molécule. La teneur en soufre peut être par exemple de 20 à 45% en poids.

Ces composés présentent de bonnes propriétés antiusure et extrême-pression, et peuvent être avantageusement utilisés comme additifs pour huiles lubrifiantes (minérales et/ou synthétiques), notamment les huiles pour engrenages et celles destinées au travail des métaux. La proportion de composé sulfuré dans l'huile peut être par exemple de 0,05 à 20% en poids, plus particulièrement de 0,2 a 5% en poids.

Les exemples suivants illustrent l'invention. L'exemple 1 est donné à titre de comparaison.

EXEMPLE 1 : (comparatif)

Synthèse du composé

[$CH_2$ = $C(CH_3)$-$CH_2$-S-$(CH_2)_3$-] $_2$ S

Première étape :

Synthèse du méthyl-2 propène-1 thiol-3

On dissout 125,7 g (1,65 mole-g) de thiourée dans 250 $cm^3$ de triéthylène glycol porté à 75 °C, puis on ajoute progressivement 135,75 g (1,5 mole-g) de chlorure de méthallyle, en maintenant la température réactionnelle inférieure à 130 °C.

On ajoute ensuite progressivement 283,5 g (1,5 mole-g) de tétraéthylène pentamine, le thiol formé qui distille (Point d'ébullition = 40 °C) est recueilli et conservé.

Deuxième étape :

Synthèse de l'alcool monosulfuré éthylénique correspondant :

On dissout 9,6 g (0,24 mole-g) de NaOH pur dans 200 $cm^3$ d'alcool éthylique puis, après dissolution, on ajoute lentement 21,1 g (0,24 mole-g) du thiol préparé dans la première étape, en maintenant la température réactionnelle en dessous de 40 °C: puis on porte le mélange à 50 °C, on maintient cette température pendant 0,5 heure, puis on refroidit à 20 °C.

On ajoute 18, 9 g(0,2 mole-g) de chloro-3 propanol-1 puis le mélange est porté à la température du reflux, on maintient cette température pendant 6 heures, on refroidit et on filtre pour éliminer le NaCl formé.

La solution alcoolique filtrée est diluée avec 1000 $cm^3$ d'eau, l'alcool soufré est extrait avec du benzène et lavé à l'eau: la solution organique récupérée est séchée sur $Na_2SO_4$ anhydre, filtrée, puis evaporée sous pression réduite. L'alcool insaturé soufré obtenu (24 g) répond aux caractéristiques analytiques suivantes :

| C % Masse | | H % Masse | | S % Masse | |
|---|---|---|---|---|---|
| Trouvé | Théorie | Trouvé | Théorie | Trouvé | Théorie |
| 57,81 | 57,51 | 9,74 | 9,58 | 22,12 | 21,95 |

Les analyses infra-rouge et RMN $^{13}C$ confirment la structure chimique du produit attendu :

$CH_2 = C(CH_3)-CH_2-S-(CH_2)_3-OH$

Troisième étape :

Synthèse du dérivé chloré correspondant à l'alcool soufré éthylènique :

20 g (0,137 mole-g) d'alcool soufré éthylénique obtenu dans la deuxième étape sont dissous dans 200 $cm^3$ de chloroforme; on ajoute ensuite lentement 8,16 g (0,685 mole-g) de $SOCl_2$, puis le mélange est porté à la température du reflux; on rajoute encore la même quantité de $SOCl_2$, puis on laisse à ébullition pendant deux heures.

Après refroidissement, le solvant est éliminée par évaporation sous pression réduite: on recueille 22,2g de produit répondant aux caractéristiques analytiques suivantes :

| C % Masse | | H % Masse | | S % Masse | | Cl % Masse | |
|---|---|---|---|---|---|---|---|
| Trouvé | Théorie | Trouvé | Théorie | Trouvé | Théorie | Trouvé | Théorie |
| 50,81 | 51,04 | 7,98 | 7,90 | 19,31 | 19,48 | 21,94 | 21,57 |

Les analyses infra-rouge et RMN $^{13}C$ confirment la structure chimique du produit attendu :

$CH_2 = C(CH_3)-CH_2-S-(CH_2)_3-Cl$

Quatrième étape :

Synthèse du dérivé monosulfuré correspondant au dérivé éthylènique monosulfuré chloré précédent :

On dissout 19,3 g (0,08 mole-g) de $Na_2S$, 9 $H_2O$ dans 100 $cm^3$ d'alcool éthylique; on porte la solution obtenue à 40 °C, puis on ajoute lentement 20 g (0,121 mole-g) du dérivé chloré précédent dissous dans 25 $cm^3$ d'alcool éthylique. Le mélange est porté à la température du reflux et est conservé à cette température

pendant 4 heures.

Après refroidissement, le mélange est filtré pour éliminer le NaCl formé, dilué avec 500 $cm^3$ d'eau et extrait au benzène: la phase organique récupérée est sèchée sur $Na_2SO_4$ anhydre, filtrée puis évaporée sous pression réduite; le produit récupéré (17g) répond aux caractéristiques analytiques suivantes :

| C % Masse | | H % Masse | | S % Masse | |
|---|---|---|---|---|---|
| Trouvé | Théorie | Trouvé | Théorie | Trouvé | Théorie |
| 58,01 | 57,90 | 9,08 | 8,96 | 32,87 | 33,15 |

Les analyses infra-rouge et RMN $^{13}$C confirment la structure chimique du produit attendu :

$[CH_2=C(CH_3)-CH_2-S-(CH_2)_3-]_2\ S$

Exemple 2 :

Synthèse du composé :

$[CH_2=C(CH_3)-CH_2-S_2-(CH_2)_3-]_2\ S$

L'expérimentation de l'exemple 1 est reprise au niveau de la seconde étape pour la préparation d'un alcool disulfuré (statistique) éthylènique, en opérant dans les conditions suivantes :

On dissout 9,6g (0,24 mole-g) de NaOH pur dans 200 $cm^3$ d'alcool éthylique puis, après dissolution, on ajoute lentement 21,1g (0,24 mole-g) du thiol préparé dans la première étape de l'exemple 1, en maintenant la température réactionnelle en dessous de 40° C; on ajoute alors 7,7 g (0,24 atome-g) de soufre élémentaire pour former le disulfure statistique, puis on porte le mélange à 50° C, on maintient cette température pendant 0,5 heure, puis on refroidit à 20°C.

Les troisième et quatrième étapes de l'expérimentation de l'exemple 1 sont ensuite poursuivies, avec les mêmes conditions opératoires et avec les mêmes proportions molaires de réactifs, pour obtenir 24 g d'un produit possèdant les caractéristiques analytiques suivantes :

| C % Masse | | H % Masse | | S % Masse | |
|---|---|---|---|---|---|
| Trouvé | Théorie | Trouvé | Théorie | Trouvé | Théorie |
| 47,87 | 47,41 | 7,54 | 7,34 | 44,79 | 45,19 |

Les analyses infra-rouge et RMN $^{13}$C confirment la structure chimique du produit attendu :

$[CH_2=C(CH_3)-CH_2-S_2-(CH_2)_3-]_2\ S$

Exemple 3 :

Synthèse du composé :

$[CH_2=C(CH_3)-CH_2-S(CH_2)_3-]_2\ S_2$

L'expérimentation de l'exemple 1 est reprise au niveau de la quatrième étape pour la préparation d'un disulfure (statistique) de sodium, en opérant dans les conditions suivantes :

On dissout 19,3 g (0,08 mole-g) de $Na_2S$, 9 $H_2O$ dans 100 $cm^3$ d'alcool éthylique; on porte la solution obtenue à 40°C, puis on ajoute 2,66 g (0,08 atome-g) de soufre élémentaire; on laisse le disulfure de sodium se former pendant 0,5 heure à cette même température, puis la même quantité de dérivé chloré préparé comme indiqué dans la troisième étape de l'exemple 1 est ajoutée : 20 g (0,121 mole-g) dans 25 $cm^3$ d'alcool éthylique. Le mélange est porté à la température du reflux et est conservé à cette température pendant 4 heures.

Après refroidissement, le mélange est filtré pour éliminer le NaCl forme, dilué avec 500 $cm^3$ d'eau et extrait au benzène ; la phase organique récupérée est sèchée sur $Na_2SO_4$ anhydre, filtrée puis évaporée

sous pression réduite; le produit récupéré (20,3 g) possède les caractéristiques analytiques suivantes :

| C % Masse | | H % Masse | | S % Masse | |
|---|---|---|---|---|---|
| Trouvé | Théorie | Trouvé | Théorie | Trouvé | Théorie |
| 52,48 | 52,13 | 8,34 | 8,07 | 39,12 | 39,80 |

Les analyses infra-rouge et RMN $^{13}C$ confirment la structure chimique du produit attendu :

$[CH_2=C(CH_3)-CH_2-S-(CH_2)_3-]_2\ S_2$

Exemple 4 :

Evaluation des propriétés extrême-pression et antiusure des additifs des exemples 1 à 3.

On a réalisé des essais mettant en évidence les propriétés extrême-pression et antiusure des additifs des exemples 1 à 3, à l'aide d'une machine 4 billes selon la procédure de l'ASTM D 2783-82 à des concentrations telles que la teneur en soufre de l'huile minérale SAE 90 soit égale à 0,4 % en masse; les resultats obtenus sont rassemblés dans le tableau 1.

TABLEAU 1

| ADDITIF DE L'EXEMPLE | QUANTITE DE S DANS L'ADDITIF (% poids) | QUANTITE D'ADDITIF DANS L'HUILE (% poids) | QUANTITE DE S DANS L'HUILE (% poids) | EXTREME-PRESSION | | | | USURE | | |
|---|---|---|---|---|---|---|---|---|---|---|
| | | | | INDICE CHARGE USURE | | CHARGE DE SOUDURE | | DIAMETRE D'EMPREINTE (mm) 1h SOUS CHARGE DE | | |
| | | | | kgf | N | kgf | N | 40 kgf 392,4 N | 60 kgf 588,6 N | 80 kgf 784,8 N |
| - | - | 0 | - | 22,50 | 220,7 | 160 | 1569,5 | 0,82 | 1,98 | 2,08 |
| 1 | 22,12 | 1,81 | 0,4 | 37,10 | 363,9 | 250 | 2452,5 | 0,64 | 0,92 | 1,95 |
| 2 | 39,12 | 0,98 | 0,4 | 65,22 | 639,8 | 400 | 3924,0 | 0,53 | 0,67 | 1,12 |
| 3 | 44,79 | 0,89 | 0,4 | 69,94 | 686,1 | 500 | 4905,0 | 0,52 | 0,62 | 1,04 |

On constate au vu de ces résultats que les additifs des exemples 2 et 3 possèdent des propriétés antiusure et extrême-pression améliorées, à concentration de soufre égale, par rapport à celle de l'additif de l'exemple comparatif 1.

EXEMPLE 5 :

Synthèse du composé (t.Bu-S-$(CH_2)_3$-$)_2$ $S_2$

Première étape :

Synthèse de l'alcool soufré correspondant t.Bu-S-$(CH_2)_3$-OH

On dissout 100 g (2,5 mole-g) de soude dans 1250 cm3 de d'alcool méthylique anhydre puis, après dissolution, on ajouta lentement 180,0 g ( 2,0 mole-g) de 2-méthyl-2 propanethiol (tert-butylmercaptan) en maintenant la température réactionnelle en dessous de 40 ° C . On porte ensuite le mélange à 50 ° C et l'on maintient cette température pendant 0,5 heure, puis on refroidit à 20 ° C .

On ajoute 189,0 g (2,0 mole-g) de 3-chloro-1 propanol puis le mélange est porté à la température du reflux, on maintient cette température pendant 6 heures, on refroidit et l'on filtre pour éliminer le NaCl formé .

La solution alcoolique filtrée recueillie est diluée avec 7500 cm3 d' eau, l'alcool soufré est extrait avec du dichlorométhane et lavé à l'eau; la solution organique récupérée est sèchée sur $Na_2SO_4$ anhydre, filtrée puis évaporée sous pression réduite pour obtenir l'alcool soufré (290,0 g) dont les caractéristiques analytiques sont les suivantes :

| C % masse | | H % masse | | S % masse | |
|---|---|---|---|---|---|
| trouvé | théorie | trouvé | théorie | trouvé | théorie |
| 56,92 | 56,71 | 10,94 | 10,88 | 21,35 | 21,63 |

Les analyses infra-rouge et RMN $^{13}C$ confirment la structure chimique du produit attendu.

Seconde étape :

Synthèse du dérivé chloré correspondant à l'alcool soufré t.Bu-S-$(CH_2)_3$-Cl

240 g (0,8 mole-g) de l'alcool soufré obtenu dans la première étape sont dissous dans 1250 cm3 de chloroforme; on ajoute ensuite lentement 49,2 g (0,41 mole-g) de $SOCl_2$ puis le mélange est porté au reflux; on ajoute encore la même quantité de $SOCl_2$, puis on laisse à ébullition pendant deux heures.

Après refroidissement, lavage à l'eau et sèchage sur $Na_2SO_4$ anhydre, le solvant est éliminé par évaporation sous pression réduite; on recueille 130 g de produit répondant aux caractéristiques analytiques suivantes:

| C % masse | | H % masse | | S % masse | | Cl % masse | |
|---|---|---|---|---|---|---|---|
| trouvé | théorie | trouvé | théorie | trouvé | théorie | trouvé | théorie |
| 50,67 | 50,43 | 9,17 | 9,07 | 19,10 | 19,23 | 21,12 | 21,27 |

Les analyses infra-rouge et RMN $^{13}C$ confirment la structure chimique du produit attendu.

Troisième étape :

Synthèse du composé polysoufré visé

On dissout 6,6 g (0,165 mole-g) de soude dans 100 cm3 d'alcool méthylique absolu puis, après dissolution, on introduit lentement 2,83 g (0,083 mole-g) d'H2S gazeux. On ajoute ensuite 2,66 g ( 0,083 at-g de soufre en fleur pour former le disulfure de sodium statistique; on porte le mélange obtenu à 40 ° C puis on ajoute lentement 25 g (0,15 mole-g) du dérivé halogèné obtenu au terme de la seconde étape; on porte le mélange à la température du reflux et on laisse réagir pendant 4 heures. Après refroidissement le mélange est filtré pour éliminer le NaCl formé, dilué avec 1 l d'eau extrait au chloroforme, séché sur $Na_2SO_4$, filtré puis évaporé sous pression réduite.

On recueille ainsi 23,0 g de produit dont les caractéristiques analytiques sont les suivantes :

| C % masse | | H % masse | | S % masse | |
|---|---|---|---|---|---|
| trouvé | théorie | trouvé | théorie | trouvé | théorie |
| 52,12 | 51,48 | 9,43 | 9,26 | 38,50 | 39,26 |

Les analyses infra-rouge et RMN $^{13}C$ confirment la structure chimique disulfure statistique attendue :

$(t.Bu-S-(CH_2)_3-)_2\ S_2$

EXEMPLE 6 :

Synthèse du composé $(t.Bu-S-(CH_2)_3-)2\ S_3$
La troisième étape de l'exemple 5 est reprise en utilisant cette fois 5,32 g (0,166 mole-g) de soufre élémentaire pour former le trisulfure statistique visé; après réaction avec 25 g du dérivé halogèné obtenu à la seconde étape de ce meme exemple et après poursuite du mode opératoire, on recueille 25,5 g de produit dont les caractéristiques analytiques sont les suivantes :

| C % masse | | H % masse | | S % masse | |
|---|---|---|---|---|---|
| trouvé | théorie | trouvé | théorie | trouvé | théorie |
| 47,01 | 46,87 | 8,75 | 8,43 | 44,14 | 44,69 |

Les analyses infra-rouge et RMN $^{13}C$ confirment la structure chimique trisulfure statistique attendue.

EXEMPLE 7 :

Synthèse du composé :

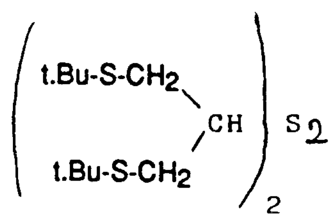

- Synthèse de l'alcool soufré correspondant .

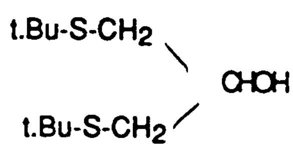

La première étape de l'exemple 5 est reprise en utilisant 12 g (0,3 mole-g) de soude, 150 cm3 d'alcool méthylique, 27 g (0,3 mole-g) de tert-butylmercaptan et 12,9 g (0,1 mole-g) de 1,3-dichloro 2-propanol; après traitements on récupère 22 g d'alcool soufré dont les caractéristiques analytiques sont les suivantes :

| C % masse | | H % masse | | S % masse | |
|---|---|---|---|---|---|
| trouvé | théorie | trouvé | théorie | trouvé | théorie |
| 56,02 | 55,88 | 10,36 | 10,23 | 26,82 | 27,12 |

Les analyses infra-rouge et RMN $^{13}C$ confirment la structure chimique du produit attendu.

- Synthèse du dérivé halogèné correspondant à l'alcool soufré .

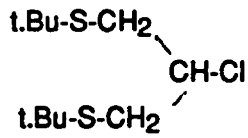

La seconde étape de l'exemple 5 est reprise en utilisant 20 g (0,0846 mole-g) d'alcool soufré précédent, 125 cm3 de chloroforme, et 11,17 g (0,093 mole-g) de $SOCl_2$; après traitements on récupère 19,5 g de produit répondant aux caractéristiques suivantes :

| C % masse | | H % masse | | S % masse | | Cl % masse | |
|---|---|---|---|---|---|---|---|
| trouvé | théorie | trouvé | théorie | trouvé | théorie | trouvé | théorie |
| 52,04 | 51,83 | 9,27 | 9,09 | 24,95 | 25,16 | 13,67 | 13,91 |

Les analyses infra-rouge et RMN $^{13}C$ confirment la structure chimique du produit attendu.

- Synthèse du composé polysoufré visé .

La troisième étape de l'exemple 5 est reprise en utilisant 3,3 g (0,0815 mole-g) de soude dans 100 cm3 d'alcool méthylique, 1,4 g (0,0425 mole-g) d'$H_2S$ , 1,36 g (0,0425 at-g) de soufre et 19 g (0,0745 mole-g) du dérivé halogèné précédent; après réaction puis traitements on récupère 17 g de produit répondant aux caractéristiques suivantes :

| C % masse | | H % masse | | S % masse | |
|---|---|---|---|---|---|
| trouvé | théorie | trouvé | théorie | trouvé | théorie |
| 52,88 | 52,53 | 9,47 | 9,22 | 37,69 | 38,25 |

Les analyses infra-rouge et RMN $^{13}C$ confirment la structure chimique disulfure statistique attendue .

EXEMPLE 8 :

Synthèse du composé $(t.Bu\text{-}S\text{-}(CH_2)_2\text{-})_2\ S_2$
L'expérimentation de l'exemple 5 est reprise, avec les mêmes proportions molaires de réactifs et les mêmes conditions opératoires, en utilisant le 2-chloroéthanol; après réactions et traitement on récupère un produit répondant aux caractéristiques suivantes :

| C % masse | | H % masse | | S % masse | |
|---|---|---|---|---|---|
| trouvé | théorie | trouvé | théorie | trouvé | théorie |
| 48,78 | 48,27 | 8,98 | 8,78 | 42,21 | 42,95 |

Les analyses infra-rouge et RMN $^{13}C$ confirment la structure chimique disulfure statistique attendue .

EXEMPLE 9

Synthèse du composé $(t.Bu\text{-}S_2\text{-}(CH_2)_3\text{-})_2\ S$

Première étape :

Synthèse de l'alcool soufré correspondant $t.Bu\text{-}S_2\text{-}(CH_2)_3\text{-}OH$
L'expérimentation de l'exemple 5 est reprise en utilisant dans la première étape 16 g (0,4 mole-g) de soude dans 250 cm3 d'alcool méthylique, 36 g (0,4 mole-g) de tert-butylmercaptan et en ajoutant 12,83 g (0.4 at-g) de soufre pour former le disulfure alcalin statistique .

La synthèse est poursuivie en utilisant 28,35 g (0.3 mole-g) de 3-chloro-1 propanol; après traitements on recueille 50 g de produit .

Seconde étape :

Synthèse du dérivé chloré correspondant à l'alcool soufré $t.Bu\text{-}S_2\text{-}(CH_2)_3\text{-}Cl$
On fait réagir, dans les conditions opératoires de la seconde étape de l'exemple 5, 45 g (0,277 mole-g) de l'alcool soufré précédent, avec 34 g de $SOCl_2$; après réaction et traitements, on récupère 51 g de produit chloré .

Troisième étape :

Synthèse du composé polysoufré visé
Dans les conditions indiquées dans l'exemple 5 on fait réagir 6,6 g (0,165 mole-g) de soude dans 100 cm3 d'alcool méthylique avec 2,81 g (0,825 mole-g) d' $H_2S$ . On ajoute ensuite 25 g ( 0,125 mole-g ) de produit chloré obtenu précédemment; après traitements on recueille 21 g de produit dont les caractéristiques sont les suivantes :

| C % masse | | H % masse | | S % masse | |
|---|---|---|---|---|---|
| trouvé | théorie | trouvé | théorie | trouvé | théorie |
| 47,53 | 46,87 | 8,94 | 8,43 | 43,53 | 44,69 |

Les analyses GPC, infra-rouge et RMN $^{13}C$ confirment la structure chimique disulfure statistique attendue, avec des impuretés du type polysulfures de di-tert-butyle en quantité très minoritaire .

EXEMPLE 10 :

Synthèse du composé $(t.Bu\text{-}S_2\text{-}(CH_2)_3\text{-})_2\ S_2$
L'expérimentation de l'exemple 9 est reprise en ajoutant dans la troisième étape 2,41g (0,075 at-g) de soufre pour former un disulfure alcalin statistique. Après réaction avec le dérivé chloré (25 g - 0,0125 mole-g) puis traitements on recueille 20 g de produit répondant aux caractéristiques suivantes :

| C % masse | | H % masse | | S % masse | |
|---|---|---|---|---|---|
| trouvé | théorie | trouvé | théorie | trouvé | théorie |
| 43,84 | 43,03 | 7,89 | 7,74 | 48,32 | 49,23 |

Les analyses GPC, infra-rouge et RMN $^{13}C$ confirment la structure chimique disulfure statistique attendue, avec des impuretés du type polysulfures de di-tert-butyle en quantité très minoritaire .

EXEMPLE 11 :

Evaluation des propriétés extrême-pression et antiusure des additifs des exemples de 5 à 10.

On a réalisé des essais mettant en évidence les propriétés extrême-pression et antiusure des additifs des exemples 5 à 11, à l'aide d'une machine 4 billes selon les procédures ASTM D 2783-82 Eet NF E 48.617 (Méthode PEUGEOT-RENAULT N° 1078), à des concentrations en additifs telles que la teneur en soufre de l'huile minérale utilisée SAE 90 soit égale à 0.4% en masse; les résultats obtenus sont rassemblés dans le tableau 2 dans lequel on a rappelé les résultats obtenus avec l'additif de l'exemple comparatif 1.

On peut constater, au vu des résultats obtenus, que les additifs des exemples 5 à 10 possedent, à concentration de soufre égale, des propriétés extrêmes-pression et antiusure bien supérieures à celles de l'additif de l'exemple comparatif 1 dans lequel le soufre ne se trouve qu'à l'état de monosulfure.

TABLEAU 2

| ADDITIF DE L'EXEMPLE | QUANTITE DE S DANS L'ADDITIF ( % masse ) | QUANTITE D'ADDITIF DANS L'HUILE ( % masse ) | QUANTITE DE S DANS L'HUILE ( % masse ) | EXTREME-PRESSION: INDICE CHARGE USURE Kgf | EXTREME-PRESSION: INDICE CHARGE USURE N | EXTREME-PRESSION: CHARGE DE SOUDURE Kgf | EXTREME-PRESSION: CHARGE DE SOUDURE N | USURE: DIAMETRE D'EMPREINTE ( mm ) 1 h SOUS CHARGE DE 40 Kgf 392.4 N | USURE: DIAMETRE D'EMPREINTE ( mm ) 1 h SOUS CHARGE DE 60 Kgf 588.6 N | USURE: DIAMETRE D'EMPREINTE ( mm ) 1 h SOUS CHARGE DE 80 Kgf 784.8 N |
|---|---|---|---|---|---|---|---|---|---|---|
| - | - | 0 | - | 22.50 | 220.7 | 160 | 1569.5 | 0.82 | 1.98 | 2.08 |
| 1 | 22.12 | 1.81 | 0.4 | 37.10 | 363.9 | 250 | 2452.5 | 0.64 | 0.92 | 1.95 |
| 5 | 38.50 | 1.04 | " | 41.80 | 410.0 | 315 | 3090.2 | 0.59 | 0.78 | 1.35 |
| 6 | 44.14 | 0.91 | " | 58.20 | 570.9 | 400 | 3924.0 | 0.54 | 0.72 | 1.00 |
| 7 | 37.69 | 1.06 | " | 43.20 | 423.8 | 315 | 3090.2 | 0.52 | 0.66 | 1.07 |
| 8 | 42.21 | 0.95 | " | 42.10 | 413.0 | 315 | 3090.2 | 0.55 | 0.77 | 1.43 |
| 9 | 43.53 | 0.92 | " | 45.70 | 448.3 | 315 | 3090.2 | 0.51 | 0.63 | 1.04 |
| 10 | 48.32 | 0.83 | " | 57.40 | 563.1 | 400 | 3924.0 | 0.53 | 0.68 | 1.01 |

**Revendications**

1. Un composé polysulfuré caractérisé en ce qu'il répond à la formule générale :

$\{[R\text{-}S_x\text{-}(CH_2)_n\text{-}]_z\ CH_{3-z}\ \text{-}(CH_2)_p\}_2\ S_y$

dans laquelle R représente un radical hydrocarboné monovalent, ou un radical hydrocarboné fontionnalisé monovalent, de 1 à 30 atomes de carbone ; x est un nombre au moins égal à 1 ; z est un nombre entier égal à 1 ou 2; lorsque z = 1, n est un nombre entier de 0 à 3, et p est un nombre entier de 0 à 3, avec n + p de 0 à 3 ; lorsque z = 2, n est un nombre entier de 1 à 3 et p un nombre entier de 0 à 3 ; et y est un nombre au moins égal à 1, au moins un des nombres x et y étant strictement supérieur à 1.

2. Composé selon la revendication 1, caractérisé en ce que R représente un radical alkyle de là 10 atomes de carbone ou un radical alkényle de 2 à 10 atomes de carbone; x a une valeur moyenne de 1 à 3; z est égal à 1, avec n + p de 1 à 3, et y a une valeur moyenne de 1 à 3.

3. Composé selon la revendication 2, caractérisé en ce que R représente un radical tertiobutyle ou un radical méthallyle.

4. Composé selon la revendication 3, caractérisé en ce qu'il répond à l'une des formules

$[t.Bu\text{-}S\text{-}(CH_2)_2\text{-}]_2\ S_2$ ; $[t.Bu\text{-}S\text{-}(CH_2)_3\text{-}]_2\ S_2$ ;
$[t.Bu\text{-}S\text{-}(CH_2)_2\text{-}]_2\ S_3$ ; $[t.Bu\text{-}S\text{-}(CH_2)_3\text{-}]_2\ S_3$ ;
$[t.Bu\text{-}S_2\text{-}(CH_2)2\text{-}]_2\ S$ ; $[t.Bu\text{-}S_2\text{-}(CH_2)_3\text{-}]_2\ S$ ;
$[t.Bu\text{-}S_2\text{-}(CH_2)_2\text{-}]_2\ S_2$ ; $[t.Bu\text{-}S_2\text{-}(CH_2)_3\text{-}]_2\ S_2$ ;
$[t.Bu\text{-}S_2\text{-}(CH_2)_2\text{-}]_2\ S_3$ ; $[t.Bu\text{-}S_2\text{-}(CH_2)_3\text{-}]_2\ S_3$ ;
$[t.Bu\text{-}S_3\text{-}(CH_2)_2\text{-}]_2\ S$ ; $[t.Bu\text{-}S_3\text{-}(CH_2)_3\text{-}]_2\ S$ ;
$[t.Bu\text{-}S_3\text{-}(CH_2)_2\text{-}]_2\ S_2$ ; $[t\ .Bu\text{-}S_3\text{-}(CH_2)_3\text{-}]_2\ S_2$ ;
$[t.Bu\text{-}S_3\text{-}(CH_2)_2\text{-}]_2\ S_3$ ; $[t.Bu\text{-}S_3\text{-}(CH_2)_3\text{-}]_2\ S_3$ ;
[méthallyl-S-$(CH_2)_2$-$]_2$ $S_2$ ; [méthallyl-S-$(CH_2)_3$-$]_2$ $S_2$ ;
[méthallyl-S-$(CH_2)_2$-$]_2$ $S_3$ ; [méthallyl-S-$(CH_2)_3$-$]_2$ $S_3$ ;
[méthallyl-$S_2$-$(CH_2)_2$-$]_2$ S ; [méthallyl-$S_2$-$(CH_2)_3$-$]_2$ S ;
[méthallyl-$S_2$-$(CH_2)_2$-$]_2$ $S_2$ ; [méthallyl-$S_2$-$(CH_2)_3$-$]_2$ $S_2$ ;
[méthallyl-$S_2$-$(CH_2)_2$-$]_2$ $S_3$ ; [méthallyl-$S_2$-$(CH_2)_3$-$]_2$ $S_3$ ;
[méthallyl-$S_3$-$(CH_2)_2$-$]_2$ S ; [méthallyl-$S_3$-$(CH_2)_3$-$]_2$ S ;
[méthallyl-$S_3$-$(CH_2)_2$-$]_2$ $S_2$ ; [méthallyl-$S_3$-$(CH_2)_3$-$]_2$ $S_2$ ;
[méthallyl-$S_3$-$(CH_2)_2$-$]_2$ $S_3$ et [méthallyl-$S_3$-$(CH_2)_3$-$]_2$ $S_3$ ;

5. Composé selon la revendication 1, caractérisé en ce que R représente un radical alkyle de 1 à 10 atomes de carbone ou un radical alkényle de 2 à 10 atomes de carbone; x a une valeur moyenne de 1 à 3; z est égal à 2, avec n égal à 1 et p égal à zéro; et y a une valeur moyenne de 1 à 3.

6. Composé selon la revendication 5, caractérisé en ce que R représente un radical tertiobutyle ou un radical méthallyle.

7. Composé selon la revendication 6, caractérisé en ce qu'il répond à l'une des formules

$[(t.Bu\text{-}S\text{-}CH_2\text{-})_2\ CH\text{-}]_2\ S_2$ ; $[(t.Bu\text{-}S\text{-}CH_2\text{-})_2\ CH\text{-}]_2\ S_3$ ;
$[(t.Bu\text{-}S_2\text{-}CH_2\text{-})_2\ CH\text{-}]_2\ S$ ; $[(t.Bu\text{-}S_2\text{-}CH_2\text{-})_2\ CH\text{-}]_2\ S_2$ ;
$[(t.Bu\text{-}S_2\text{-}CH_2\text{-})_2\ CH\text{-}]_2\ S_3$ ; $[(t.Bu\text{-}S_3\text{-}CH_2\text{-})_2\ CH\text{-}]_2\ S_3$ ;
$[\ (t.Bu\text{-}S_3\text{-}CH_2\text{-})_2\ CH\text{-}]_2\ S_2$ $[(t.Bu\text{-}S_3\text{-}CH_2\text{-})_2\ CH\text{-}]_2\ S_3$ ;
[(méthallyl-S-$CH_2$-$)_2$ CH-$]_2$ $S_2$ [(méthallyl-S-$CH_2$-$)_2$ CH-$]_2$ $S_3$ ;
[(méthallyl-$S_2$-$CH_2$-$)_2$ CH-$]_2$ S [(méthallyl-$S_2$-$CH_2$-$)_2$ CH-$]_2$ $S_2$ ;
[(méthallyl-$S_2$-$CH_2$-$)_2$ CH-$]_2$ $S_3$ [(méthallyl-$S_3$-$CH_2$-$)_2$ CH-$]_2$ S ;
[(méthallyl-$S_3$-$CH_2$-$)_2$ CH-$]_2$ $S_2$ -[(méthallyl-$S_3$-$CH_2$-$)_2$ CH-$]_2$ $S_3$ .

8. Utilisation d'un composé selon l'une des revendications 1 à 7 comme additif pour huiles lubrifiantes.

9. Utilisation selon la revendication 8, dans laquelle la concentration en ledit composé est de 0,05 à 20% en poids par rapport à ladite huile lubrifiante.

## Claims

1. Polysulphide compound, characterized in that it complies with the general formula:

$$\{[R\text{-}S_x\text{-}(CH_2)_n\text{-}]_z\ CH_{3-z}\ \text{-}(CH_2)_p\ \text{-}\}_2\ S_y$$

in which R represents a monovalent hydrocarbon radical with 1 to 30 carbon atoms, which is optionally functionalized, x is a number equal to or greater than 1, z is an integer equal to 1 or 2; when z = 1, n is an integer from 0 to 3 and p an integer from 0 to 3, with n + p an integer of 0 to 3, when z = 2, n is an integer from 1 to 3 and p an integer from 0 to 3, and y is a number equal to or greater than 1, at least one of the numbers x and y being strictly higher than 1.

2. Compound according to claim 1, characterized in that R represents an alkyl radical with 1 to 10 carbon atoms or an alkenyl radical with 2 to 10 carbon atoms, x has a mean value of 1 to 3, z is equal to 1, with n + p being 1 to 3 and y having a mean value of 1 to 3.

3. Compound according to claim 2, characterized in that R represents a tert. butyl or methallyl radical.

4. Compound according to claim 3, characterized in that it complies with one of the formulas:

$[t.Bu\text{-}S\text{-}(CH_2)_2\text{-}]_2\ S_2$ ; $[t.Bu\text{-}S\text{-}(CH_2)_3\text{-}]_2\ S_2$
$[t.Bu\text{-}S\text{-}(CH_2)_2\text{-}]_2\ S_3$ ; $[t.Bu\text{-}S\text{-}(CH_2)_3\text{-}]_2\ S_3$
$[t.Bu\text{-}S_2\text{-}(CH_2)_2\text{-}]_2\ S$ ; $[t.Bu\text{-}S_2\text{-}(CH_2)_3\text{-}]_2\ S$
$[t.Bu\text{-}S_2\text{-}(CH_2)_2\text{-}]_2\ S_2$ ; $[t.Bu\text{-}S_2\text{-}(CH_2)_3\text{-}]_2\ S_2$
$[t.Bu\text{-}S_2\text{-}(CH_2)_2\text{-}]_2\ S_3$ ; $[t.Bu\text{-}S_2\text{-}(CH_2)_3\text{-}]_2\ S_3$
$[t.Bu\text{-}S_3\text{-}(CH_2)_2\text{-}]_2\ S$ ; $[t.Bu\text{-}S_3\text{-}(CH_2)_3\text{-}]_2\ S$
$[t.Bu\text{-}S_3\text{-}(CH_2)_2\text{-}]_2\ S_2$ ; $[t.Bu\text{-}S_3\text{-}(CH_2)_3\text{-}]_2\ S_2$
$[t.Bu\text{-}S_3\text{-}(CH_2)_2\text{-}]_2\ S_3$ ; $[t.Bu\text{-}S_3\text{-}(CH_2)_3\text{-}]_2\ S_3$
$[methallyl\text{-}S\text{-}(CH_2)_2\text{-}]_2\ S_2$ ; $[methallyl\text{-}S\text{-}(CH_2)_3\text{-}]_2\ S_2$
$[methallyl\text{-}S\text{-}(CH_2)_2\text{-}]_2\ S_3$ ; $[methallyl\text{-}S\text{-}(CH_2)_3\text{-}]_2\ S_3$
$[methallyl\text{-}S_2\text{-}(CH_2)_2\text{-}]_2\ S$ ; $[methallyl\text{-}S_2\text{-}(CH_2)_3\text{-}]_2\ S$
$[methallyl\text{-}S_2\text{-}(CH_2)_2\text{-}]_2\ S_2$ ; $[methallyl\text{-}S_2\text{-}(CH_2)_3\text{-}]_2\ S_2$
$[methallyl\text{-}S_2\text{-}(CH_2)_2\text{-}]_2\ S_3$ ; $[methallyl\text{-}S_2\text{-}(CH_2)_3\text{-}]_2\ S_3$
$[methallyl\text{-}S_3\text{-}(CH_2)_2\text{-}]_2\ S$ ; $[methallyl\text{-}S_3\text{-}(CH_2)_3\text{-}]_2\ S$
$[methallyl\text{-}S_3\text{-}(CH_2)_2\text{-}]_2\ S_2$ ; $[methallyl\text{-}S_3\text{-}(CH_2)_3\text{-}]_2\ S_2$
$[methallyl\text{-}S_3\text{-}(CH_2)_2\text{-}]_2\ S_3$ and $[methallyl\text{-}S_3\text{-}(CH_2)_3\text{-}]_2\ S_3$

5. Compound according to claim 1, characterized in that R represents an alkyl radical with 1 to 10 carbon atoms or an alkenyl radical with 2 to 10 carbon atoms, x has a mean value of 1 to 3, z is equal to 2, with n equal to 1 and p equal to zero and y has a mean value of 1 to 3.

6. Compound according to claim 5, characterized in that R represents a tert. butyl or methallyl radical.

7. Compound according to claim 6, characterized in that it complies with one of the following formulas:

$[(t.Bu\text{-}S\text{-}CH_2\text{-})_2\ CH\text{-}]_2\ S_2$ ; $[(t.Bu\text{-}S\text{-}CH_2\text{-})_2\ CH\text{-}]_2\ S_3$
$[(t.Bu\text{-}S_2\text{-}CH_2\text{-})_2\ CH\text{-}]_2\ S$ ; $[(t.Bu\text{-}S_2\text{-}CH_2\text{-})_2\ CH\text{-}]_2\ S_2$
$[(t.Bu\text{-}S_2\text{-}CH_2\text{-})_2\ CH\text{-}]_2\ S_3$ ; $[(t.Bu\text{-}S_3\text{-}CH_2\text{-})_2\ CH\text{-}]_2\ S_3$
$[(t.Bu\text{-}S_3\text{-}CH_2\text{-})_2\ CH\text{-}]_2\ S_2$ ; $[(t.Bu\text{-}S_3\text{-}CH_2\text{-})_2\ CH\text{-}]_2\ S_3$
$[(methallyl\text{-}S\text{-}CH_2\text{-})_2\ CH\text{-}]_2\ S_2$ ; $[(methallyl\text{-}S\text{-}CH_2\text{-})_2\ CH\text{-}]_2\ S_3$
$[(methallyl\text{-}S_2\text{-}CH_2\text{-})_2\ CH\text{-}]_2\ S$ ; $[(methallyl\text{-}S_2\text{-}CH_2\text{-})_2\ CH\text{-}]_2\ S_2$
$[(methallyl\text{-}S_2\text{-}CH_2\text{-})_2\ CH\text{-}]_2\ S_3$ ; $[(methallyl\text{-}S_3\text{-}CH_2\text{-})_2\ CH\text{-}]_2\ S$
$[(methallyl\text{-}S_3\text{-}CH_2\text{-})_2\ CH\text{-}]_2\ S_2$ ; $[(methallyl\text{-}S_3\text{-}CH_2\text{-})_2\ CH\text{-}]_2\ S_3$

8. Use of a compound according to any one of the claims 1 to 7 as an additive for lubricating oils.

9. Use according to claim 8, wherein the concentration of said compound is 0.05 to 20% by weight, based on said lubricating oil.

**Patentansprüche**

1. Polysulfidische Verbindung, dadurch gekennzeichnet, daß sie der allgemeinen Formel:

$\{[R\text{-}S_x\text{-}(CH_2)_n]_z\ CH_{3-z}\text{-}(CH_2)_p\}_2 S_y$

entspricht, worin R einen einwertigen Kohlenwasserstoffrest oder einen funktionalisierten einwertigen Kohlenwasserstoffrest mit 1 bis 30 Kohlenstoffatomen darstellt; x eine Zahl von wenigstens gleich 1 ist; z eine ganze Zahl von gleich 1 oder 2 ist; wenn z = 1, n eine ganze Zahl von 0 bis 3 und p eine ganze Zahl von 0 bis 3 ist, wobei n + p von 0 bis 3 beträgt; wenn z = 2, n eine ganze Zahl von 1 bis 3 und p eine ganze Zahl von 0 bis 3 ist; und y eine Zahl von wenigstens gleich 1 ist, wobei wenigstens eine der Zahlen x und y streng über 1 liegt.

2. Verbindung nach Anspruch 1, dadurch gekennzeichnet, daß R einen Alkylrest mit 1 bis 10 Kohlenstoffatomen oder einen Alkylenrest mit 2 bis 10 Kohlenstoffatomon darstellt; x einen mittleren Wert von 1 bis 3 besitzt; z gleich 1 ist, wobei n + p 1 bis 3 beträgt und y einen mittleren Wert von 1 bis 3 besitzt.

3. Verbindung nach Anspruch 2, dadurch gekennzeichnet, daß R einen t-Butyl- oder einen Methallylrest darstellt.

4. Verbindung nach Anspruch 3, dadurch gekennzeichnet, daß sie einer der allgemeinen Formeln

$[t\text{-}Bu\text{-}S\text{-}(CH_2)_2\text{-}]_2 S_2$; $[t\text{-}Bu\text{-}S\text{-}(CH_2)_3\text{-}]_2 S_2$;
$[t\text{-}Bu\text{-}S\text{-}(CH_2)_2\text{-}]_2 S_3$; $[t\text{-}Bu\text{-}S\text{-}(CH_2)_3\text{-}]_2 S_3$;
$[t\text{-}Bu\text{-}S_2\text{-}(CH_2)_2\text{-}]_2 S$; $[t\text{-}Bu\text{-}S_2\text{-}(CH_2)_3\text{-}]_2 S$;
$[t\text{-}Bu\text{-}S_2\text{-}(CH_2)_2\text{-}]_2 S_2$; $[t\text{-}Bu\text{-}S_2\text{-}(CH_2)_3\text{-}]_2 S_2$;
$[t\text{-}Bu\text{-}S_2\text{-}(CH_2)_2\text{-}]_2 S_3$; $[t\text{-}Bu\text{-}S_2\text{-}(CH_2)_3\text{-}]_2 S_3$;
$[t\text{-}Bu\text{-}S_3\text{-}(CH_2)_2\text{-}]_2 S$; $[t\text{-}Bu\text{-}S_3\text{-}(CH_2)_3\text{-}]_2 S$;
$[t\text{-}Bu\text{-}S_3\text{-}(CH_2)_2\text{-}]_2 S_2$; $[t\text{-}Bu\text{-}S_3\text{-}(CH_2)_3\text{-}]_2 S_2$;
$[t\text{-}Bu\text{-}S_3\text{-}(CH_2)_2\text{-}]_2 S_3$; $[t\text{-}Bu\text{-}S_3\text{-}(CH_2)_3\text{-}]_2 S_3$;
$[Methallyl\text{-}S\text{-}(CH_2)_2\text{-}]_2 S_2$; $[Methallyl\text{-}S\text{-}(CH_2)_3\text{-}]_2 S_2$;
$[Methallyl\text{-}S\text{-}(CH_2)_2\text{-}]_2 S_3$; $[Methallyl\text{-}S\text{-}(CH_2)_3\text{-}]_2 S_3$;
$[Methallyl\text{-}S_2\text{-}(CH_2)_2\text{-}]_2 S$; $[Methallyl\text{-}S_2\text{-}(CH_2)_3\text{-}]_2 S$;
$[Methallyl\text{-}S_2\text{-}(CH_2)_2\text{-}]_2 S_2$; $[Methallyl\text{-}S_2\text{-}(CH_2)_3\text{-}]_2 S_2$;
$[Methallyl\text{-}S_2\text{-}(CH_2)_2\text{-}]_2 S_3$; $[Methallyl\text{-}S_2\text{-}(CH_2)_3\text{-}]_2 S_3$;
$[Methallyl\text{-}S_3\text{-}(CH_2)_2\text{-}]_2 S$; $[Methallyl\text{-}S_3\text{-}(CH_2)_3\text{-}]_2 S$;
$[Methallyl\text{-}S_3\text{-}(CH_2)_2\text{-}]_2 S_2$; $[Methallyl\text{-}S_3\text{-}(CH_2)_3\text{-}]_2 S_2$;
$[Methallyl\text{-}S_3\text{-}(CH_2)_2\text{-}]_2 S_3$ und $[Methallyl\text{-}S_3\text{-}(CH_2)_3\text{-}]_2 S_3$

entspricht.

5. Verbindung nach Anspruch 1, dadurch gekennzeichnet, daß R einen Alkylrest mit 1 bis 10 Kohlenstoffatomen oder einen Alkylenrest mit 7 bis 10 Kohlenstoffatomen darstellt; x einen mittleren Wert von 1 bis 3 besitzt; z gleich 2 ist, wobei n gleich 1 und p gleich Null ist und y einen mittleren Wert von 1 bis 3 besitzt.

6. Verbindung nach Anspruch 5, dadurch gekennzeichnet, daß R einen t-Butyl- oder einen Methallylrest darstellt.

7. Verbindung nach Anspruch 6, dadurch gekennzeichnet, daß sie einer der allgemeinen Formeln:

$[(t\text{-}Bu\text{-}S\text{-}CH_2\text{-})_2\ CH\text{-}]_2 S_2$; $[(t\text{-}Bu\text{-}S\text{-}CH_2\text{-})_2\ CH\text{-}]_2 S_3$;
$[(t\text{-}Bu\text{-}S_2\text{-}CH_2\text{-})_2\ CH\text{-}]_2 S$; $[(t\text{-}Bu\text{-}S_2\text{-}CH_2\text{-})_2\ CH\text{-}]_2 S_2$;

$[(t\text{-}Bu\text{-}S_2\text{-}CH_2\text{-})_2\ CH\text{-}]_2S_3$; $[(t\text{-}Bu\text{-}S_3\text{-}CH_2\text{-})_2\ CH\text{-}]_2S_3$;
$[(t\text{-}Bu\text{-}S_3\text{-}CH_2\text{-})_2\ CH\text{-}]_2S_2$ $[(t\text{-}Bu\text{-}S_3\text{-}CH_2\text{-})_2\ CH\text{-}]_2S_3$;
$[(Methallyl\text{-}S\text{-}CH_2\text{-})_2\ CH\text{-}]_2S_2$; $[(Methallyl\text{-}S\text{-}CH_2\text{-})_2\ CH\text{-}]_2S_3$;
$[(Methallyl\text{-}S_2\text{-}CH_2\text{-})_2\ CH\text{-}]_2S$; $[(Methallyl\text{-}S_2\text{-}CH_2\text{-})_2\ CH\text{-}]_2S_2$;
$[(Methallyl\text{-}S_2\text{-}CH_2\text{-})_2\ CH\text{-}]_2S_3$; $[(Methallyl\text{-}S_3\text{-}CH_2\text{-})_2\ CH\text{-}]_2S$;
$[(Methallyl\text{-}S_3\text{-}CH_2\text{-})_2\ CH\text{-}]_2S_2$; u.$[(Methallyl\text{-}S_3\text{-}CH_2\text{-})_2\ CH\text{-}]_2S_3$;

entspricht.

**8.** Verwendung einer Verbindung nach einem der Ansprüche 1 bis 7 als Additiv für Schmiermittel.

**9.** Verwendung nach Anspruch 8, worin die Konzentration der Verbindung bezüglich des Schmiermittels 0.05 bis 20 Gew.% beträgt.